# EUROPEAN PATENT SPECIFICATION

(11) **EP 1 094 843 B1**
(45) Date of publication and mention of the grant of the patent: **01.10.2003**
(21) Application number: 99933960.9
(22) Date of filing: 13.07.1999
(51) Int. Cl.: A61K 47/48, A61K 41/00, A61K 49/00

(54) **TARGETED SITE SPECIFIC DRUG DELIVERY COMPOSITIONS AND USES**
ZIELGERICHTE ORTSSPEZIFISCHE ARZNEIMITTELZUSAMMENSETZUNGEN UND VERWENDUNGEN
COMPOSITIONS DESTINEES A L'ADMINISTRATION CIBLEE SPECIFIQUE D'UN SITE DE MEDICAMENTS ET UTILISATIONS

(30) Priority: 13.07.1998 US 114399; 17.07.1998 US 118168
(43) Date of publication of application: 02.05.2001
(73) Proprietor: UNIVERSITY OF NEBRASKA BOARD OF REGENTS, Lincoln, NE 68598 (US)
(72) Inventor: PORTER, Thomas, R., Omaha, NE 68118 (US); IVERSEN, Patrick, L., Corvallis, Oregon 97330 (US); MEYER, Gary D., Athens, OH 45701 (US)
(74) Representative: VOSSIUS & PARTNER
(86) International application number: US9915801
(87) International publication number: WO00002588

(56) References cited:
- WO-A-96/39197
- WO-A-97/33474
- WO-A-98/00172
- WO-A-98/18498
- US-A- 5 567 415
- PORTER T R ET AL: "THE EFFECT OF MICROBUBBLE GAS COMPOSITION AND EXTERNAL ULTRASOUND FREQUENCY ON THE NON-INVASIVE ENHANCEMENT OF ANTISENSE OLIGONUCLEOTIDE DELIVERY TO THE VASCULAR WALL IN PIGS" CIRCULATION,US,DALLAS, TX, vol. 96, no. 8, page L-401 XP000198974 ISSN: 0009-7322
- PORTER T R ET AL: "VISUALLY DISCERNIBLE MYOCARDIAL ECHOCARDIOGRAPHIC CONTRAST AFTER INTRAVENOUS INJECTION OF SONICATED DEXTROSE ALBUMIN MICROBUBBLES CONTAINING HIGH MOLECULAR WEIGHT, LESS SOLUBLE GASES" JOURNAL OF THE AMERICAN COLLEGE OF CARDIOLOGY,XX,XX, vol. 25, no. 2, page 509-515 XP000590866 ISSN: 0735-1097

## Description

### Field of the Invention

The present invention relates to a pharmaceutical composition and method for delivery of bioactive substances. The methods and compositions of the invention can be used to achieve site specific delivery of a biologically active substance. This specificity allows for lower drug doses and improved efficacy, particularly for agents such as oligonucleotides which are typically plagued with problems in achieving therapeutic concentrations in targeted organs.

### Background of the Invention

Drug delivery techniques are employed in drug therapy to augment drug availability, to reduce drug dose, and consequently to reduce drug-induced side effects. These techniques serve to control, regulate, and target the release of drugs in the body. Ongoing goals of drug delivery have included less frequent drug administration, maintenance of constant and continuous therapeutic levels of a drug in the systemic circulation or at a specific target organ site, reduction in undesirable side effects, and reduction in the amount and dose concentration required to realize the desired therapeutic benefits. Finally, methods to noninvasively target the delivery of a desired drug to a target organ are desired.

To date, drug delivery systems have included drug carriers based upon proteins, polysaccharides, synthetic polymers, erythrocytes, DNA and liposomes. New generation biologicals such as monoclonal antibodies, gene therapy vectors, anti-cancer drugs such as taxol, viral based drugs, and oligonucleotides (ODN) and polynucleotides have presented several problems with regard to delivery. In fact, drug delivery may be the primary hurdle to achieving mainstream therapeutic use of these agents, whose initial potential seemed unlimited but whose therapeutic parameters have prevented realization of full benefit.

The use of synthetic oligodeoxyribonucleotides (ODN's), chemically modified to confer nuclease resistance, represents a fundamentally different approach to drug therapy. The most common applications to date employ antisense ODN's with sequences complementary to a specific targeted mRNA sequence. An antisense oligonucleotide approach to therapy involves a simple and specific drug design concept, in which the ODN causes a mechanistic intervention in the processes of translation or an earlier processing event. The advantage of this approach is the potential for gene-specific actions, which should be reflected in a relatively low dose and minimal non-targeted side effects. However, phosphorothioate oligonucleotides, currently the most commonly employed analogs in biological studies, frequently exhibit non-specific binding to proteins. In general, the primary potential advantages of the antisense approach (low dose and minimal side effects) have fallen short of expectations.

Drug delivery of oligonucleotides and polynucleotides has focused on two key challenges: transfection of oligonucleotides into cells and alteration of distribution of oligonucleotides *in vivo.* With respect to transfection, biological approaches to improve *in vitro* cellular uptake have included the use of vehicles such as liposomes and viral vectors such as reconstituted viruses and pseudovirions. Methods to improve biodistribution have focused on such agents as cationic lipids, which are postulated to increase cellular uptake of drugs due to the positively charged lipid attraction to the negatively charged surfaces of most cells.

Cationic liposomes have been reported to enhance gene transfer into vascular cells *in* vivo when administered by catheter (Muller, D.W. *et al., Circ. Res.* **75**(6):103949, 1994). Cationic lipid DNA complexes have also been reported to result in effective gene transfer into mouse lungs after intratracheal administration. Asialoglycoprotein poly(L)-lysine complexes have met with limited success, as has complexation with Sendai virus coat protein containing liposomes. Toxicity and biodistribution have remained significant issues.

From the foregoing, it can be seen that more effective targeted drug delivery systems for delivery of biologics, particularly poly- and oligonucleotides, are needed for these drags to achieve their full potential.

Several references describe the use of gas-filled microbubbles as contrast agents. In U.S. Patent No. 5,567,415, Porter describes microbubbles prepared by sonicating a dextrose-albumin suspension while introducing a perfluorocarbon gas. Porter *et al. (J. Am. Coll. Cordial,* **25**(2):509, 1995) teaches gas-containing microbubbles which are exposed to air, nitrogen, helium or SF₆ by (a) sonication in air followed by (b) continuous mixing with the selected gas in a syringe. Other references have described microbubbles for delivery of therapeutics. Unger (WO 96/39197) teaches drug delivery via microbubbles encapsulated in a fluorinated amphiphilic compound. The encapsulated gases may include fluorocarbons, nitrogen, oxygen, or air. In a typical preparation, the microbubbles are formed by flushing the head space over an aqueous suspension of fluorinated amphiphilic compound with a fluorocarbon and/or nitrogen, and shaking the container. Porter *et al*. *(Circulation* **96**(8):L401, 1997) discloses microbubbles for containing either room air or a fluorocarbon, for delivery of oligonucleotides. Porter and Iversen (WO 98/00172) compare protein-encapsulated microbubbles containing different gases, i.e. nitrogen, helium, and sulfur hexafluoride. The microbubbles are prepared by sonication in room air, followed by mixing with the respective gas on a hematology rocker. Marsden (WO 98/18498) describes targeted ultrasound contrast agents for use in diagnostic imaging and/or therapy. The reference teaches that the agents may contain any of a large number of gases; gases actually employed in the 35 working examples are perfluorocarbons, SF₆, or air. Porter et *al.* (WO 97/33474) teaches the delivery of drugs by conjugation to microbubbles, in combination with ultrasound. The PESDA (perfluorocarbon exposed sonicated dextrose/albumin) microbubbles were prepared by hand-agitation with perfluorocarbon followed by sonication in air.

### Summary of the Invention

In one aspect, the invention provides a use as claimed in claim 1 for delivering a biological agent to a specific tissue site. The preparation of the microbubbles comprises the steps of (a) forming an aqueous suspension of a plurality of protein-encapsulated, insoluble gas-containing microbubbles, where the biological agent is conjugated to the protein, under conditions which produce an enhanced oxygen content and a lower partial pressure of N₂ within the microbubbles than that obtained via room air sonication; and (b) administering the suspension to an animal, such that the protein directs the microbubble-conjugated agent to sites of bioprocessing of the protein and, upon dissipation of the microbubble, releases the biological agent. Preferably, the microbubbles are formed in an N₂-depleted, and more preferably N₂-free, environment, such as oxygen.

In preferred embodiments, the protein encapsulating the microbubbles is selected from the group consisting of albumin, human gamma globulin, human apotransferrin, β-lactose and urease; albumin is particularly preferred. The insoluble gas is preferably a perfluorocarbon, such as perfluoromethane, perfluoroethane, perfluoropropane, perfluorobutane, or perfluoropentane; perfluoropropane and perfluorobutane, are particularly preferred. The tissue site may be, for example, the liver or the kidney of the animal.

Preferred biological agents to be conjugated to the protein include polynucleotides, oligonucleotides, nucleotides, and ribozymes, as well as naproxen, piroxicam, warfarin, furosemide, phenylbutazone, valproic acid, sulfisoxazole, ceftriaxone, and miconazole. In selected embodiments, the biological agent is a polynucleotide, e.g. an antisense oligonucleotide, an antigens oligonucleotide, an oligonucleotide probe, a vector, a viral vector, or a plasmid. Preferred classes of polynucleotides include phosphorothioate oligonucleotides.

The invention also provides a related use as claimed in claim 3 for delivering a biological agent to a specific tissue site, comprising the steps of (a) forming an aqueous suspension of protein-encapsulated, insoluble gas-containing microbubbles, as described above, where the biological agent is conjugated to the protein; (b) administering the suspension to an animal; and (c) exposing the tissue site to ultrasound. The microbubbles are formed under conditions which produce an enhanced oxygen content and a lower partial pressure of N₂ within the microbubble than that obtained via room air sonication. The microbubbles are thus preferably formed in an N₂-depleted, and more preferably N₂-free, environment, such as oxygen.

In practicing any of these methods, the microbubble suspension is preferably formed by carrying out the following steps: (a) diluting an aqueous albumin solution comprising about 2% to about 10% by weight, preferably about 5 % by weight, of human serum albumin by about two-fold to about eight-fold, preferably about three-fold, with an aqueous dextrose solution comprising 5% to 50% by weight, preferably about 5% by weight, of dextrose; (b) agitating the solution with the insoluble gas; and (c) exposing the solution to a sonication horn, preferably in an N₂-depleted environment, to create cavitation at particulate sites in the solution, thereby generating stable microbubbles from about 0.1 to 10 microns in diameter. Preferably, the N₂-depleted environment of step (b) is an N₂-free environment, such as oxygen, which is blown into the interface between the sonicating horn and the solution.

In another aspect, the invention provides a microbubble composition as claimed in claim 16 useful for ultrasonic imaging, comprising an aqueous suspension of a plurality of protein-encapsulated insoluble gas-containing microbubbles, wherein the partial pressure of N₂ in the microbubbles is less than the partial pressure of N₂ in room air sonicated microbubbles. In a related aspect, the invention provides a composition useful for delivery of a biological agent to a target site, comprising an aqueous suspension of such microbubbles having a biological agent conjugated to the protein. Preferably, the microbubbles are N₂-free. The preferred size of the microbubbles is 0.1 to 10 microns in diameter.

In these compositions, the protein is preferably selected from albumin, human gamma globulin, human apotransferrin, β-lactose and urease; albumin is particularly preferred. The insoluble gas is preferably a perfluorocarbon, such as perfluoromethane, perfluoroethane, perfluoropropane, perfluorobutane, and perfluoropentane; perfluorobutane and perfluoropropane are particularly preferred. Biological agents suitable for conjugation to the protein encapsulating the microbubbles include oligonucleotides, polynucleotides, and ribozymes, as well as naproxin, piroxicam, warfarin, furosemide, phenylbutazone, valproic acid, sulfisoxazole, ceftriaxone, and miconazole. Oligonucleotides are particularly preferred.

The invention also provides, in particular, a use as claimed in claim 13 of preventing carotid artery stenosis formation in an animal undergoing vascular trauma. Preferably, this method comprises the steps of (a) forming an aqueous suspension of albumin-encapsulated, insoluble gas-containing microbubbles, where the gas filling the microbubbles is N₂-depleced or, preferably, N₂-free, and the albumin is conjugated to a nucleic acid biological agent which inhibits the expression of a gene encoding a regulatory enzyme which mediates smooth muscle proliferation, and (b) administering the suspension to the animal. A preferred method further includes the step of (c) exposing the site of the trauma to ultrasound to promote release of the biological agent. The inhibited gene is preferably c-myc or c-myb.

As noted above, these compositions and uses can be used to significantly reduce the effective dosages of biological agents, increasing the therapeutic index and improving bioavailability. This reduction in dose can in turn reduce drug cytotoxicity and side effects. Furthermore, the invention can enhance the effectiveness of other plasma-bound drugs such as heparin, diltiazem, lidocaine, propanolol, cyclosporin, and chemotherapeutic agents which require blood pool activation. For example, it is shown herein that the anticoagulant properties of heparin can be dramatically enhanced by first combining the medicament with orosomucoid-labeled perfluorocarbon-exposed sonicated dextrose/albumin, and then administering the combination intravenously.

The conjugates are designed for parenteral administration as an aqueous suspension. After administration, and timed to coincide with arrival of the injected bolus at the site of interest, energy may be administered in the form of sound waves to cause the microbubbles to cavitate; the agent is then released and delivered to the organ or other site or interest. Conjugation of the biologic with albumin- or other protein-encapsulated microbubbles can allow for targeted delivery of the biologic to specific tissues, including those which traditionally interact with the protein.

Improved gas-filled microbubbles are achieved by forming the microbubbles in the presence of an N₂-depleted or N₂-free environment. Such an environment produces microbubbles which are smaller but more stable in venous and arterial blood. These smaller microbubbles produce greater diagnostic ultrasound contrast in the myocardium and can carry drugs to these regions with greater efficacy in therapeutic embodiments.

The invention uses agents and methods traditionally used in diagnostic ultrasound imaging, and as such provides a means for visualization of the therapeutic at its target site for delivery of the therapeutic.

These and other objects and features of the invention will become more fully apparent when the following detailed description of the invention is read in conjunction with the accompanying drawings.

### Description of the Figures

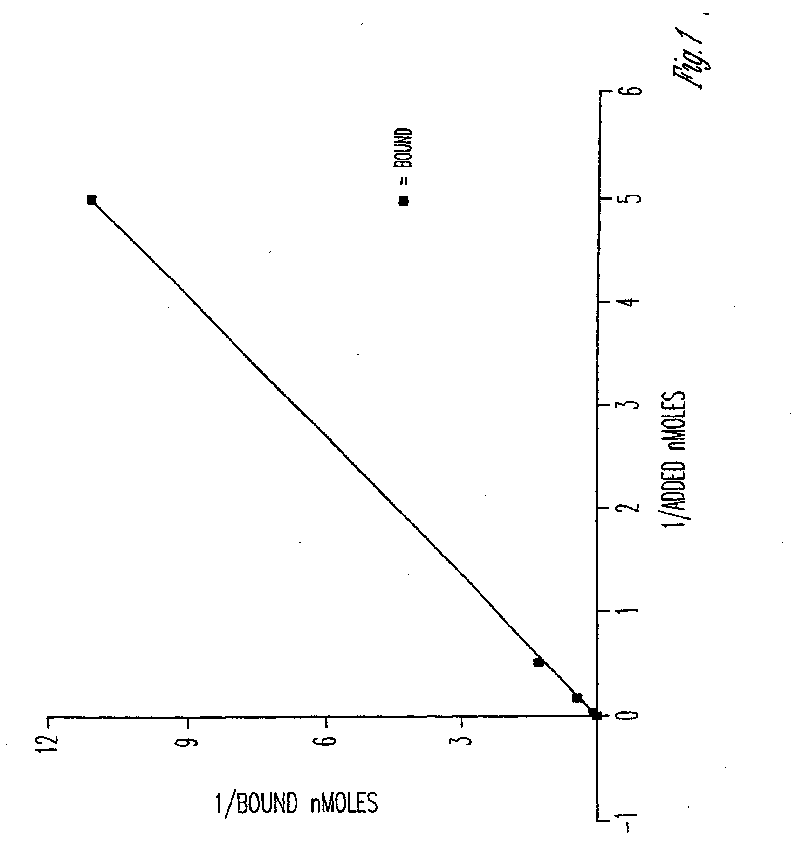

Figure 1 is a Lineweaver-Burke plot of the binding data for PESDA (perfluorocarbon exposed sonicated dextroselalbumin) microbubbles with PS-ODN. The equilibrium dissociation constant Kₘ (calculated for the 7 concentrations which were run in duplicate; see Example 5) for the binding to the microbubbles was 1.76 X 10⁻⁵M. This value is nearly within the range of 3.7 - 4.8 X 10⁻⁵ M reported for binding a 15mer PS-ODN with sequence 5'd(AAC GTT GAG GGG CAT)-3'; SEQ ID NO: 1) to human serum albumin in solution (Srinivasan, S.K. *et al.,* "Characterization of Binding Sites, Extent of Binding, and Drug Interactions of Oligonucleotides with Albumin", *Antisense Res. Devel.* **5**:131, 1995).

### Detailed Description of the Invention

### I. Therapeutic Uses of Ultrasound

Ultrasonic imaging has long been used as a diagnostic tool to aid in therapeutic procedures. It is based on the principle that waves of sound energy can be focused upon an area of interest and reflected to produce an image. Generally, an ultrasonic transducer is placed on a body surface overlying the area to be imaged, and ultrasonic energy, produced by generating and receiving sound waves, is transmitted. The ultrasonic energy is reflected back to the transducer where it is translated into an ultrasonic image. The amount and characteristics of the reflected energy depend upon the acoustic properties of the tissues. Contrast agents which are echogenic are preferably used to create ultrasonic energy in the area of interest and improve the imaging received. For a discussion of contrast echographic instrumentation, see DeJong, "Acoustic Properties of Ultrasound Contrast Agents", Cip-Gegevens Koninklijke Bibliotheek, Denhag (1993), pp. 120 *et seq.*

Contrast echocardiography has been used to delineate intracardiac structures, assess valvular competence, and demonstrate intracardiac shunts. Myocardial contrast echocardiography (MCE) has been used to measure coronary blood flow reserve in humans. MCE has been found to be a safe and useful technique for evaluating relative changes in myocardial perfusion and delineating areas at risk.

Ultrasonic vibration has also been used at therapeutic levels in the medical field to increase the absorption of various medicaments. For example, JP Kokai number 52-115591 discloses that percutaneous absorption of a medicament is enhanced by ultrasonic vibration. U.S. Patent Nos. 4,953,565 and 5,007,438 also disclose a technique of percutaneous absorption of medicaments by the aid of ultrasonic vibration. U.S. Patent No. 5,315,998 discloses a booster for drug therapy comprising microbubbles in combination ultrasonic energy to allow the medicament to diffuse and penetrate. This reference discloses the use of therapeutic levels of ultrasound for up to 20 minutes, in contrast to certain embodiments of the present invention, which use diagnostic levels of ultrasound with exposure for much shorter time periods to promote release of conjugated bioactive agents.

In accordance with the present invention, traditional diagnostic ultrasound therapy contrast agents can be used to bind and then release therapeutic agents at specifically designated sites of interest, thereby altering drug distribution. This objective can be accomplished with the contrast agent alone and without the use of any diagnostic ultrasound. It is also demonstrated herein that such contrast agents can be used in combination with traditional diagnostic ultrasound therapy (e.g. including peak negative pressures in the range of 0.1 to 3.5 MPa, and transmit frequencies in the range of 1.0 to 40 MHz) to promote release of therapeutic agents at the site of interest. In a preferred embodiment of this method, low frequency ultrasound is used, namely frequencies less than 1 MHz, most preferably from about 10 kHz to about 40 kHz.

### II. Therapeutic Compositions

The pharmaceutical composition of the invention comprises a liquid suspension, preferably an aqueous suspension, of microbubbles containing a blood-insoluble gas, preferably having a diameter of 0.1 to 10 microns. The microbubbles are formed by entrapping microbubbles of such a gas into a liquid. The microbubbles may contain various blood-insoluble gases such as fluorocarbons or sulfur hexafluoride gas. Generally, any insoluble gas which is nontoxic and gaseous at body temperature can be used. The gas must also form stable microbubbles, having an average size of between about 0.1 and 10 microns in diameter, when the pharmaceutical composition is sonicated to form microbubbles. Microbubbles having a mean diameter in this range are suitable for transpulmonary passage, and are stable enough to prevent significant diffusion of gases within the microbubble following intravenous injection and during transit to the target site.

The insoluble gas must also have a diffusion coefficient and blood solubility lower than nitrogen or oxygen, which diffuse in the internal atmosphere of the blood vessel. Examples of useful gases are fluorocarbon gases and sulfur hexafluoride. Generally, perfluorocarbon gases, such as perfluoromethane, perfluoroethane, perfluoropropane, perfluorobutane, and perfluoropentane, are preferred. Of these gases, perfluoropropane and perfluorobutane are especially preferred because of their demonstrated safety for intraocular injection in humans (see e.g. Wong and Thompson, *Ophthalmology* **95**:609-613).

The gaseous microbubbles are stabilized by a filmogenic protein coating. Suitable proteins include naturally occurring proteins such as albumin, human gamma globulin, human apotransferrin, β-lactose and urease. The invention preferably employs a naturally occurring protein, but synthetic proteins may also be used. Particularly preferred is human serum albumin. This protein is easily metabolized within the body and has been widely used as a contrast agent.

It is preferred to use an aqueous solution containing a mixture of a pharmaceutically accepted saccharide, e.g. dextrose, in combination with the earlier described protein. Exemplary of other saccharides suitable for use in the invention are monosaccharides (having the formula C₆H₁₂O₆ , such as the hexose sugars, dextrose, fructose or mixtures thereof), disaccharides (having the formula C₁₂H₂₂O₁₁, such as sucrose, lactose or maltose or mixtures thereof), or polysaccharides (e.g. soluble starches having the formula (C₆H₁₀O₅)ₙ, where n is a whole number between 20 and about 200, such as amylase or dextran or mixtures thereof). The saccharide, however, is not essential to achieve the results of the invention.

The therapeutic agent of the invention is formulated in a pharmaceutically effective dosage form for peripheral administration to the host, optionally in conjunction with ultrasound therapy. Generally such host is a human host, although other mammalian hosts such as canine or equine can also be subject to this therapy.

### III. Preparation of the Microbubble Compositions

In a preferred embodiment, the composition of the invention is formulated from a mixture of commercially available albumin (human), U.S.P. solution (generally supplied as a 5% or 25% by weight sterile aqueous solution) and commercially available dextrose (U.S.P. for intravenous administration). In a most preferred embodiment, the mixture comprises a two-fold to eight-fold dilution of a 2% to 10% by weight solution of human serum albumin with a 5% to 50% by weight solution of dextrose.

The microbubbles are formed by sonication, typically with a sonicating horn, at room temperature and ambient pressure. Such sonication causes cavitation within the dextrose/albumin solution at sites of particulate matter or gas in the fluid. These cavitation sites eventually resonate and produce small microbubbles which are non-collapsing and stable. During sonication, the solution is perfused with the insoluble gas, preferably a perfluorocarbon gas, for about 80 seconds. In general, sonication conditions which produce concentrations of greater than about 4x10⁸ M of microbubbles between about 0.1 to 10 µm, and preferably 5-6 µm in diameter, are preferred. The resulting microbubbles are concentrated at room temperature for at least about 120 ± 5 minutes, whereupon the excess solution settles in the sonicating syringe.

In an alternate method of preparation, as described in Example 2, 15 ± 2 ml of sonicated dextrose/albumin is hand agitated with 8 ± 2 ml of perfluorocarbon gas prior to sonication. Sonication then proceeds for 80 ± 5 seconds.

A third method of preparation, designed to improve the affinity of the microbubble for many plasma-bound drugs, includes the addition of 5 to 30 mg of orosomucoid (α-acid glycoprotein) to the albumin/dextrose solution prior to sonication.

In accordance with the invention, the microbubbles are preferably formed in an N₂-depleted, preferably N₂-free environment, typically by introducing an N₂-depleted (in comparison to room air) or N₂-free gas into the interface between the sonicating horn and the solution. As discussed further below, microbubbles formed in this way are significantly smaller than those formed in room air. These smaller microbubbles are more stable and result in improved delivery of therapeutics and diagnostics.

The microbubbles are then incubated with the biologically active agent so that the medicament becomes conjugated (typically by non-covalent binding) with the protein-coated microbubble. Binding studies of oligonucleotides to albumin-coated microbubbles are described in Examples 4-7. These studies showed, for example, that the binding was saturable, and thus nonspecific interactions are limited (Example 6). In Example 7, it is demonstrated that filmogenic proteins in the form of microbubbles, as conventionally used in contrast agents, retain their ability to bind compounds when the microbubbles are filled with an insoluble gas. This finding is contrary to earlier reports that, in such microbubble contrast agents, the protein sphere was comprised of denatured protein. For example, see U.S. Patent No. 4,572,203, U.S. Patent No. 4,718,433 and U.S. Patent No. 4,774,958. As demonstrated herein, when an insoluble gas, e.g. a perfluorocarbon, rather than air, is used for the microbubble, the protein retains its binding activity. This effect is very likely due to the fact that much of the sonication energy is absorbed by the insoluble gas. Thus the protein, such as albumin, can bind to biologically active medicaments to form a microbubble/agent conjugate. Air filled microbubbles, on the other hand, are less likely to retain their binding capabilities.

### IV. Therapeutic Agents

Therapeutic agents useful in the present invention are selected via their ability to bind with the filmogenic protein, and vice versa. For example, if the filmogenic protein is albumin, the therapeutic can include oligonucleotides, polynucleotides, or ribozymes, all of which can bind with albumin and as such can form a conjugation with the microbubble. A list of drugs which bind to albumin at site 1, which is confirmed to remain intact in the present compositions and methods, follows:

| Drug | % Albumin Binding | Drug Class |
|---|---|---|
| Naproxen | 99.7 | NSAID^{a} |
| Piroxicam | 99.3 | NSAID^{a} |
| Warfarin | 99.0 | Anticoagulant |
| Furosemide | 98.8 | Loop diuretic |
| Phenylbutazone | 96.1 | NSAID^{a} |
| Valproic Acid | 93.0 | Antiepileptic |
| Sulfisoxazole | 91.4 | Sulfonamide antibiotic |
| Ceftriaxone | 90-95^{b} | Third generation cephalosporin antibiotic |
| Miconazole | 90.7-93.1^{b} | Antifungal |
| Phenytoin | 89.0 | Antiepileptic |

| | | |
|---|---|---|
| ^{a}Nonsteroidal anti inflammatory drug | | |
| ^{b}Represents patient-to-patient variability | | |

If orosomucoid is added to the dextrose/albumin, then the list of drugs which could be potentiated also includes erythromycin (antibiotic), lidocaine (antiarrhythmic), meperidine (analgesic), methadone (analgesic), verapamil and diltiazem (antianginals), cyclosporin (an immunosuppressant), propanolol (antihypertensive and antianginal), and quinidine (antiarrhythmic).

Other drugs which bind with albumin, particularly at site 1, would also be useful in this embodiment and can be ascertained by those of skill in the art through drug interaction and pharmacology texts standard to the art, such as "Drug Information" AHFS 1999 (American Society of Health System) or "Drug Facts and Comparisons", published by Berney Olin and updated every quarter. Assays for determination of appropriate protein-biologic agent combinations are disclosed therein and can be used to test any combination for its ability to work with the method of the invention.

As demonstrated below, the invention is of particular relevance for oligonucleotide and polynucleotide therapy, as the primary hurdle to effective antisense, anti-gene, probe diagnostics or even gene therapy employing viral or plasmid nucleotide delivery has been the ability of the therapeutic to reach the target site at high enough concentrations to achieve a therapeutic effect. The invention is particularly useful for delivery of nucleotide sequences in the form of gene therapy vectors, diagnostic nucleotide probes, or in antisense or anti-gene type strategies to ultimately alter or detect gene expression in target cells.

Oligonucleotides used in the present invention can include one or more modifications of the nucleic acid bases, sugar moieties, internucleoside phosphate linkages, or combinations of modifications at these sites. The internucleoside phosphate linkages may be e.g. phosphorothioate, phosphoramidate, methylphosphonate, phosphorodithioate or combinations of such similar linkages (to produce mixed-backbone modified oligonucleotides). Other oligonucleotide analogs include morpholino oligonucleotides, peptide nucleic acids, 2'-O-allyl or 2'-O-alkyl modified oligonucleotides, and N3' → P5' phosphoramidates. In a preferred embodiment the oligonucleotide is a phosphorothioate oligonucleotide.

Any of the known methods for oligonucleotide synthesis can be used to prepare the oligonucleotides. They are most conveniently prepared using any of the commercially available, automated nucleic acid synthesizers, such as Applied Biosystems, Inc., DNA synthesizer (Model 380B). using phosphoramidate chemistry, according to manufacturer protocols. Phosphorothioate oligonucleotides may be synthesized and purified according to the methods described in Stek and Zon (*J*. *Chromatography,* **326**:263-280) and in Applied Biosystems DNA Synthesizer User Bulletin, Models 380A-380B-381A-391-EP, December 1989. The ODN is introduced to cells by methods which are known to those of skill in the art. See, e.g., Iversen, 1991, *"In vivo* Studies with Phosphorothioate Oligonucleotides: Pharmacokinetics Prologue", *Anticancer Drug Des.* **6**:531-8.

### V. Delivery Methods

In preferred methods for practicing the delivery therapy of the invention, a pharmaceutical liquid agent of the invention is introduced into a mammalian host by intravenous injection, intravenously (i.v. infusion), percutaneously or intramuscularly, preferably near the target site of activity of the agent. Therapeutic sites can include such sites as the location of a specific tumor, location of a specific infection, an organ which due to differential gene activation expresses a particular gene product, the site of an injury or thrombosis, a site for further processing and distribution of the therapeutic, etc.

### A. Without Ultrasound

According to one embodiment of the invention, protein (e.g. albumin) coated microbubbles of insoluble (e.g. perfluorocarbon) gas have been shown to form stable conjugates with oligonucleotides (see Examples 4-6). The ODN conjugated bubbles are introduced to the animal, whereby the protein coating can direct the conjugated agent to sites of interaction. Ultimately, as the bubble dissipates, the agent is released at the tissue site. The inventors have shown that application of ultrasound is not necessary for the targeted delivery of biologics to sites of bioprocessing of the protein coating. This is shown in Example 9, where ODN-conjugated microbubbles were directed to the liver and kidney, and were effective in altering the metabolism of hexobarbital. The protein traffics the microbubble and conjugate to sites of processing, and, as the bubbles dissipate, the oligonucleotide or other biologic is released to interact at the site, allowing for a fraction of the biological agent to achieve the same biological effect as compared to conventional administration.

Generally the target site is selected based upon the bioprocessing of the filmogenic protein. For example, the kidneys and liver take up albumin, and albumin microbubbles can be used to specifically direct the administration of conjugated bioactive agents to these areas. This biodistribution is demonstrated in Example 9, as noted above. The metabolism and bioprocessing of other filmogenic proteins is described in standard pharmacological texts such as "Basic and Clinical Pharmacology", 7th ed., Bertram G. Katzung, ed., Appleton & Lange, 1997.

### B. With Ultrasound

In one embodiment of the invention, a diagnostic ultrasound field is introduced when the injected bolus reaches the target site. As demonstrated in Example 8, exposure of PS-ODN-labeled perfluorocarbon-containing microbubbles to diagnostic ultrasound did not alter the integrity of the PS-ODN, but did release the ODN from its albumin binding.

Application of ultrasound can be accomplished in various ways. For example, an ultrasound transducer can be placed directly above the target site. Conventional ultrasonic devices can supply an ultrasonic signal of 20 kHz to several MHz. The signal is generally applied from about 3 to about 5 MHz for diagnostic ultrasound and at levels of less than 1 MHz, preferably about 20 kHz, for the therapeutic ultrasound. The energy provided by the ultrasound will cause the microbubble to release the agent at the therapeutic site or into the blood pool. This method also allows for visualization as the bolus enters the ultrasound field.

Alternatively, the ultrasound transducer can be placed over a site in the blood pool which will permit constant exposure of the microbubbles as they pass through circulation. This procedure enhances the systemic effects of certain drugs, such as heparin, as shown in Example 13.

Examples of placement sites where ultrasound enhancement may be used include the kidneys, heart chambers, aorta, or vena cavae. Example 12 describes targeted sonification of the kidney combined with delivery of labeled ODN-conjugated microbubbles. Sonification limited to a single kidney resulted in a significant increase in ODN detected at this site. Described further in Section VI, below, is the use of targeted sonification in inhibition of arterial restenosis, by administration of antisense to c-myc accompanied by sonification of the affected area.

In another embodiment, the injectate can be monitored or timed until it reaches the target area. The contrast agent may be used alone to time the travel from administration to therapeutic site. After administration of the therapeutic, low frequency (20 kHz to about 2 MHz) ultrasound is introduced at the target site after the appropriate time interval, using a suitable Doppler or ultrasound echo apparatus, so that the field of ultrasound encompasses the target site and medicament is released from the microbubble. The time period will generally vary according to the organ of interest, as well as the injection site.

### VI. Inhibition of Restenosis

### A. Background

Several investigators have shown that neointimal hyperplasia occurs after vascular balloon injury as a result of smooth muscle cell migration and proliferation (Austin, G.E. *et al.,* "Intimal Proliferation of Smooth Muscle Cells as an Explanation for Recurrent Coronary Artery Stenosis After Percutaneous Transluminal Coronary Angioplasty", *J Am. Coll. Cardiol.* **6**:369-75, 1985; Libby, P. *et al.,* "A Cascade Model for Restenosis: A Special Case of Atherosclerosis Progression", *Circulation* **86**:III47-III52, 1992; Clowes, A.W. *et al.,* "Regulation of Smooth Muscle Cell Growth in Injured Artery", *J. Cardiovasc. Pharmacol.* **14**:S12-S15, 1989). This neointimal formation plays a role in the angiographic observation of restenosis after both balloon injury and intravascular stenting (Mintz, G.S. *et al.,* "Intravascular Ultrasound Insights into Mechanisms of Stenosis Formation And Restenosis", *Cardiol.-Clin.* **15**:1:17-29, 1997). Synthetic antisense oligodeoxynucleotides (ODN) that inhibit synthesis of the protooncogenes responsible for vascular smooth muscle proliferation have successfully inhibited stenosis formation following coronary or carotid injury (Shi, Y. *et al.,* "Transcatheter Delivery of *C-Myc* Antisense Oligomers Reduces Neointimal Formation in a Porcine Model of Coronary Artery Balloon Injury", *Circulation* **90**:944-51, 1994; Morishita, R. et al., "Intimal Hyperplasia After Vascular Injury is Inhibited by Antisense CDK2 Kinase Oligonucleotides", *J. Clin. Invest.* **93**:1458-64, 1994). To this point, such treatment has required direct intravascular or periadventitial delivery. The present inventors have demonstrated that ODN (specifically, to *c-myc* and *c-myb)* bind to perfluorocarbon exposed sonicated dextrose/albumin (PESDA) microbubbles (Porter, T.R. *et al.,* "Interaction of Diagnostic Ultrasound with Synthetic Oligonucleotide-Labeled Perfluorocarbon-Exposed Sonicated Dextrose Microbubbles", *J. Ultrasound. Med.* **15**:577-584, 1996). Subsequent work showed that transcutaneous low frequency ultrasound increases the deposition of the ODN into vessels contained within the field of insonification (Porter, T.R. *et al.,* "The Effect of Microbubble Gas Composition and External Ultrasound Frequency on the Non-Invasive Enhancement of Antisense Oligonucleotide Delivery to the Vascular Wall in Pigs", *Circulation Suppl.* 2249, 1997). The study described in Example 14 was carried out to determine whether this enhanced vascular deposition with low frequency ultrasound and intravenously injected ODN to *c-myc* bound to PESDA microbubbles could inhibit neointimal formation following balloon injury of the carotid artery.

Previous methods of ODN delivery following arterial injury in previous studies have required either direct intraarterial delivery or periadventitial application (Shi, Y. *et al.,* 1994, cited above; Morishita, R. *et al.,* 1994, cited above; Bennett, M.R, *et al.,* "Effect of Phosphorothioated Oligonucleotides on Neointimal Formation in the Rat Carotid Artery", *Arterioscler. Thromb. Vasc. Biol.* **17**:2326-32, 1997).

### B. Present Study

Example 14 describes inhibition of carotid artery neointimal formation in pigs using transcutaneous ultrasound and an intravenous microbubble delivery system, as described herein, containing antisense to the *c-myc* protooncogene. The region over the right carotid artery was insonified before and after each injection for a total of six minutes both immediately after balloon dilatation and on day three. As demonstrated in the Example, ultrasound-targeted deposition of intravenously administered ODN bound to PESDA microbubbles inhibited stenosis formation by inhibiting intimal hyperplasia as well as creating a significantly smaller intimal thickness to lumen diameter ratio.

In addition to being non-invasive, the ultrasound targeted approach is advantageous because it can be repeated at various time intervals following injury. Periadventitial application of antisense to *c-myc* has suppressed medial replication, but this suppression is no longer evident at four days following carotid injury in rats (Bennett M.R. *et al.,* 1997, cited above). In the present study, as described in Example 12, a second 0.5 milligram dose of ODN bound to PESDA was administered intravenously three days following injury. Both decreased intimal hyperplasia and a larger vessel lumen were observed at 30 days in the ODN-PESDA group. It is unknown whether the larger lumen size was due to vessel growth in the ODN-PESDA group or shrinkage in the control groups, since pre-balloon injury IVUS data was not available.

Vessel enlargement has been shown to be a critical factor in determining the degree of stenosis formation following balloon injury, more important than the amount of intimal hyperplasia that occurs (Kakuta, T. *et al.,* "Differences in Compensatory Vessel Enlargement, Not Intimal Formation, Account for Restenosis After Angioplasty in the Hypercholesterolemic Rabbit Model", *Circulation* **89**:2809-15, 1994). The larger lumen area in the subjects treated with ultrasound targeted delivery may indicate that an important effect of ODN to *c-myc* is to prevent inadequate compensatory enlargement in response to atherosclerosis. One way ODN could produce lumen enlargement is by preventing *c-myc*-mediated cell migration out of the media, a process that has been inhibited by direct application of the antisense to *c-myc* (Bennett M.R. *et al.,* 1997, cited above; Biro, S. *et al.,* "Inhibitory Effects of Antisense Oligonucleotides Targeting *c-myc* mRNA on Smooth Muscle Cell Proliferation and Migration", *Proc. Natl. Acad. Sci. USA* **90**:654-58, 1993). Another mechanism whereby ODN could alter vessel size is by inhibiting c-myc's ability to stimulate endothelin-1 production, a potent vasoconstrictor and mitogenic substance (Shichiri. M. *et al.,* "Biphasic Regulation of the Preproendothelin-1 Gene by *c-myc", Endocrinology* **138**(11):4584-90, 1997).

This study confirms that the enhanced uptake of ODN bound to PESDA microbubbles in the presence of ultrasound has an important effect on stenosis formation following carotid balloon injury, and demonstrates the physiologic effectiveness of ultrasound and microbubbles as a delivery system.

Ultrasound has been shown to enhance gene expression in cultured HeLa cells when the oligonucleotides are delivered on other carrier systems like cationic liposomes (Unger, E.C. *et al.,* "Ultrasound Enhances Gene Expression of Liposomal Transfection", *Invest. Radial.* **32**:723-27, 1997). The mechanism for this enhanced cellular uptake in the presence of ultrasound has been postulated to be cavitation-induced bilayer disordering of the cell membrane (Mitragotri, S. *et al.,* "Transdermal Drug Delivery Using Low-Frequency Sonophoresis", *Pharmaceutical Research* **13**:411-20, 1996). Therefore, microbubbles may have an inherent advantage over other carrier systems by virtue of their ability to lower this cavitation threshold (Holland, C.K., *et al.,* "Thresholds for Transient Cavitation Produced by Pulsed Ultrasound in a Controlled Nuclei Environment", *J. Acoust. Soc. Am.* **88**: 2059-69, 1990). If cavitation is the mechanism for enhanced uptake of ODN, both the presence of microbubbles and the lower frequency of ultrasound used in this study (20 kHz) may have improved uptake.

In conclusion, intravenous ODN-PESDA and transcutaneous low frequency ultrasound have been shown to inhibit carotid stenosis formation in a manner similar to direct application of antisense to the vessel wall following balloon injury. These data demonstrate that ultrasound and a microbubble delivery system containing ODN may be a powerful, non-invasive, method to inhibit stenosis formation following balloon angioplasty or intravascular stenting.

### VII. Advantages of Microbubble Preparation in an N₂-Depleted Environment

Microbubbles containing an albumin shell permit rapid diffusion of soluble gases across their membranes. Perfluorocarbon containing-microbubbles survive longer than room air-containing microbubbles with the same membrane, due to the slow rate of diffusion of the higher molecular weight gas and its insolubility in blood.

Perfluorocarbon containing-microbubbles can, however, still contain a significant quantity of room air gas when prepared by room air sonication (i.e., without any modification of the sonicating environment). In this sense, "insoluble gas-containing", as used herein, refers to microbubbles which are formed by agitation with a blood-insoluble gas, e.g. a perfluorocarbon, but are expected to also contain other gases present during sonication. Room air-sonicated microbubbles may thus be affected by the concentration gradient that exists across the albumin membrane.

Mathematical models have suggested that microbubbles containing insoluble gases persist longer if tissue and blood contain nitrogen (Burkard, M.E. *et al.,* "Oxygen Transport to Tissue by Persistent Bubbles: Theory and Simulations", *J. Appl. Physiol.* 2874-8, 1994.). In the absence of blood nitrogen (i.e., oxygenated blood), nitrogen from within the PCMB would be expected to diffuse out of the PCMB, reducing their size.

Accordingly, as shown in Example 10, PCMB exposed to 100% oxygenated arterial blood were significantly smaller in size than PCMB exposed to room air blood (see Table 4, Example 10). This *in vitro* study confirms that oxygenated blood reduced the size of perfluorocarbon-containing MB, in comparison to normal blood, as nitrogen diffused out of the microbubbles into the oxygenated blood. However, the oxygenated blood did not completely destroy the perfluorocarbon-containing microbubbles, as has been shown with pure room air-containing albumin microbubbles, due to rapid diffusion of the soluble gases out of the microbubble (Wible J. Jr. *et al.*, "Effects of Inspired Gas on the Efficacy of Albunex® in Dogs", *Circulation* **88**(suppl):1-401 (1993)).

Since surface tension and absorptive pressures are increased as microbubble diameter decreases, it was hypothesized that the videointensity produced by intravenous PCMB would also be affected by alterations in nitrogen and oxygen concentration inside and outside the microbubble. It was postulated that by enhancing microbubble oxygen content (thus lowering the partial pressure of N₂ within the microbubble), microbubble survival in blood could be prolonged. This is accomplished by forming the microbubbles in an N₂-depleted or N₂-free environment.

As used herein, the term "N₂-depleted" or "nitrogen depleted" refers to an N₂ content which is less than that of room air, such that the partial pressure of N₂ in the gas-filled microbubbles formed by sonication in the presence of such an environment is lower than that achieved from sonication in the presence of room air. (Room air typically contains about 75.5% N₂ by weight, or 78% by volume). Typically, the N₂-depleted environment has a higher oxygen content than that of room air. An N₂-depleted environment is "N₂-free" when it is substantially free of N₂, e.g. commercially supplied pure oxygen.

Accordingly, the microbubbles are sonicated in an N₂-depleted or N₂-free environment, typically by blowing an N₂-depleted or N₂-free gas (such as oxygen) into the interface between the sonicating horn and the solution. In this case, the "insoluble gas-containing" microbubbles contain the insoluble gas (e.g. perfluorocarbon) and are expected to contain some quantity of the N₂-depleted or N₂-free gas. (Although some small quantity of room air may enter the microbubbles even under these conditions, the gas contained in the microbubbles is considered to be substantially N₂-free.)

This modification was found to produce substantially smaller microbubbles, which were more stable in the bloodstream, resulting in improved contrast and drug delivery. Such an effect was consistently observed in the closed chest studies described in Example 11, resulting in greater myocardial contrast than PCMB sonicated in the presence of room air. Even with intermittent imaging using pulsing intervals as short as 100 milliseconds (10 Hertz imaging), visually evident myocardial contrast was still achieved with the microbubbles sonicated in an N₂-free environment.

The invention includes the use of such contrast agents in pharmaceutical compositions and application of ultrasound as a targeted delivery system. The use of an N₂-depleted or N₂-free environment in the manufacture of the contrast agents is also an improvement in the effectiveness of the contrast agent in myocardial imaging.

### EXAMPLES

The following examples are for illustration purposes only and are not intended to limit this invention in any way. It will be appreciated by those of skill in the art that numerous other protein-bioactive agent combinations can be used in the invention and are contemplated herein. For example, if the filmogenic protein is α1 acid glycoprotein, the bioactive agent could be lidocaine, inderal, or heparin.

In all the following examples, all parts and percentages are by weight unless otherwise specified, and all dilutions are by volume.

### Example 1. Phosphorothioate Oligonucleotide Synthesis

Chain extension syntheses were performed on a 1 µmole column support on an ABI Model 391 DNA synthesizer (Perkin Elmer, Foster City, CA) or provided by Lynx Therapeutics, Inc. (Hayward CA). The 1 µmol synthesis employed cyanoethyl phosphoramidites and sulfurization with tetraethylthiuram disulfide as per ABI User Bulletin 58. Radiolabeled oligonucleotides were synthesized as hydrogen phosphonate material by Glen Research (Bethesda, MD).

### Example 2. Preparation of Microbubble Suspensions

Five percent human serum albumin and five percent dextrose were obtained from commercial sources. Three parts of 5 % dextrose solution and one part 5 % human serum albumin solution (total 16 milliliters) were drawn into a 35-milliliter Monojet® syringe. Each dextrose/albumin sample was hand agitated with 8±2 milliliters of either decafluorobutane or room air, and the sample was then exposed to electromechanical sonication at 20 kilohertz for 80±5 seconds. The mean size of four consecutive samples of the perfluorocarbon-exposed sonicated dextrose/albumin (PESDA) microbubbles produced in this manner, as measured with hemocytometry, was 4.6±0.4 microns, and mean concentration, as measured by a Coulter counter, was 1.4x10⁹ bubbles/mL.

### Example 3. Preparation of Microbubble/ODN Conjugates

Uniformly ³⁵S-labeled PS-ODNs (phosphorothioate oligonucleotides), with sequences 5'-TAT GCT GTG CCG GGG TCT TCG GGC 3' (24-mer complementary to c-myb) (SEQ ID NO:2) and 5' TTAGGG 3' (SEQ ID NO:3), were incubated in a final volume of 0.5 ml with the perfluorocarbon-exposed sonicated dextrose/albumin (PESDA) microbubble solution for 30 minutes at 37°C. The solutions were allowed to stand so that the bubbles could rise to the top; samples could then be removed either from the clear solution at the bottom or from the top layer containing the microbubbles.

### Example 4. Phosphorothioate ODN Binding to Washed or Unwashed PESDA Microbubbles

To produce washed microbubbles, solutions of microbubbles as prepared in Example 1 were washed in a 1000 times volume excess of 5% dextrose, to remove albumin which was not associated with the microbubbles. The microbubbles were allowed four hours to rise. The lower solution was then removed, leaving the washed foam, which was then mixed with 0.9% sodium chloride. Albumin protein concentration in the washed microbubbles was 0.28± 0.04 mg/ml as determined by the Bradford Assay (Bradford, M. *et al.,* "A Rapid and Sensitive Method for the Quantification of Microgram Quantities of Protein Utilizing the Principle of Protein-Dye Binding," *Anal. Biochem.* **72**:248, 1976).

A radioactively labeled 24-mer PS-ODN was added to a washed or unwashed suspension of PESDA microbubbles, at a concentration of 5 nM. Radioactivity in solutions was measured with a liquid scintillation counter (model LSC7500; Beckman Instruments GmbH, Munich, Germany). Hydrocount biodegradable scintillation cocktail (5 ml) was added to 100µl samples. Samples were counted immediately after each experiment and then again 24 hours later in order to reduce the influence of chemiluminescence and quenching.

The partitioning of PS-ODN to PESDA microbubbles (top layer) and washed (albumin-free) and unwashed (albumin containing) lower layers, as counted by liquid scintillation counting, is shown in Table 1. The recovery of total radioactivity in the experiments reported in Table 1 was approximately 96%.

**Table 1:**

| **Oligonucleotide Binding To Albumin of PESDA Microbubbles in The Presence Or Absence of Free Albumin** | | | | |
|---|---|---|---|---|
| **Unwashed Bubbles (Free Albumin Present)** | | | | |
| **Oligo** | **N** | **Top (cpm/µL)** | **Bottom (cpm/mL)** | **Ratio, T/B** |
| TTAGGG | 6 | 125±6.4 | 92.3±6.4 | 1.35 |
| c-myb | 6 | 94.1±17.6 | 77.3±1.2 | 1.35 |

| **Washed Bubbles (No Free Albumin)** | | | | |
|---|---|---|---|---|
| TTAGGG | 6 | 210±10.8 | 126±8.7 | 1.67 |
| c-myb | 6 | 200.3±37.4 | 92.7±15.7 | 2.16 |

The data indicate that albumin in the unwashed solution which is not associated with the microbubble binds to the PS-ODN. Removal of non-microbubble associated albumin (washed bubbles) resulted in a increase of partitioning of the PS-ODNs with the PESDA microbubbles (ratio = 1.67 for TTAGGG PS-ODN and 2.16 for c-myb PS-ODN).

### Example 5. Binding Affinity of ODN to Washed Albumin-Coated Microbubbles

In order to evaluate the affinity of binding of PS-ODN to the washed microbubbles of Example 4, mixtures were prepared containing increasing amounts of excess non-radioactive PS-ODN as a competing ligand for binding sites. Non-radioactive PS-ODN 20-mer, with sequence 5'-d(CCC TGC TCC CCC CTG GCT CC)-3' (SEQ ID NO:4), was added to tubes containing the radioactive 24-mer in a series of increasing concentrations (0, 3.3, 10, 32.7, 94.5, 167, and 626 µM). Each suspension of bubbles was mixed by inversion and incubated at 37°C for 60 minutes.

The observed binding data are presented as a Lineweaver-Burke plot in Figure 1. The equilibrium dissociation constant Kₘ (calculated for the 7 concentrations run in duplicate) for the binding to the microbubbles was 1.76 X 10⁻⁵ M.

### Example 6. ODN-Microbubble Competitive Binding Study

Three groups of samples were prepared in triplicate as follows, and each sample was incubated for 20 minutes at room temperature.
Group A (control): 100 µl of microbubbles / 900 µL saline + 2µL unlabeled 20-mer
Group B: 100 µL of microbubbles / 900µL of saline + 2µL FITC-labeled 20-mer (final 20-mer concentration = 151 nM)
Group C: 100 µL of microbubbles / 800 µL of saline + 2µL FITC-labeled 20-mer + 100 µL unlabeled 20-mer

Flow cytometric analysis was performed using a FACStar Plus (Becton Dickinson) equipped with a 100 mW air-cooled argon laser and Lysis II acquisition and analysis software. List mode data were employed for a minimum of 10⁴ collected microbubbles, and independent analysis was done for each sample. The distribution of FITC-labeled microbubbles is provided in Table 2.

**Table 2:**

| **Distribution of Oligonucleotide Bound Microbubbles** | | | | | | |
|---|---|---|---|---|---|---|
| | Control (PS-ODN) | | 151 nM FITC PS-ODN | | +Excess Unlabeled ODN | |
| Sample # | PE | MI | PE | MI | PE | MI |
| 1 | 99.5 | 2.38 | 98.9 | 2109.8 | 97.8 | 1753.1 |
| 2 | 99.3 | 4.07 | 99.1 | 2142.3 | 98.7 | 1710.9 |
| 3 | 99.4 | 3.52 | 99.1 | 2258.5 | 99.3 | 1832.2 |
| Mean ±SE | | 3.23 ± 0.50 | | 2170 ± 46¹ | | 1765 ± 36^{1,2} |

| | | | | | | |
|---|---|---|---|---|---|---|
| PE=percent events; MI = mean intensity; SE=standard error ¹ mean is significantly different from control; P < 0.001 ² mean is significantly different from 151 nM sample; P < 0.001 | | | | | | |

The significant decrease in mean fluorescence intensity in the samples containing excess unlabeled PS-ODN indicates that the binding to microbubbles is saturable. Consequently, nonspecific interactions of PS-ODN with the microbubble surface are limited.

### Example 7. Binding of ODN to Room Air- vs. Perfluorocarbon-Containing Microbubbles

The uniformity of binding of PS-ODN to room air versus perfluorocarbon-containing sonicated dextrose/albumin microbubbles was determined by flow cytometry. A Gaussian distribution of PS-ODN to washed PESDA microbubbles (data not shown) indicated that the albumin on these microbubbles had retained its binding site for the oligonucleotide. The absence of a Gaussian distribution for washed RA-SDA microbubbles indicated that loss of albumin binding site 1 for this oligonucleotide occurred during sonication of these microbubbles. (For a discussion of albumin binding characteristics, particularly as they relate to oligonucleotides, see Kumar *et al.,* "Characterization of Binding Sites, Extent of Binding, and Drug Interactions of Oligonucleotides with Albumin" *Antisense Res. Dev.* **5**: 131-139 (1995)).

From the foregoing, it can be seen that PS-ODN binds to the albumin in PESDA microbubbles, indicating that the binding site 1 on albumin is biologically active following production of these bubbles by electromechanical sonication. This binding site affinity is lost, however, when the electromechanical sonication is performed with room air only. Further, removal of albumin not associated with PESDA microbubbles, by washing, results in a significant partitioning of the PS-ODNs with the microbubbles (Table 1). These observations demonstrate that albumin denaturation does not occur with perfluorocarbon-containing dextrose/albumin solutions during sonication, as has been suggested with sonication in the presence of air.

The retained bioactivity of albumin (especially at site 1) in PESDA microbubbles was confirmed by the affinity of binding of PS-ODN to washed PESDA microbubbles in the presence of increasing amounts of excess non-radioactive PS-ODN as a competing ligand for binding sites (Table 2). The significant decrease in mean fluorescence intensity in the samples containing excess unlabeled PS-ODN indicates the binding to microbubbles is saturable.

### Example 8. Exposure of Microbubbles with Bound Oligonucleotide to Ultrasound

A variable flow microsphere scanning chamber, similar to that described previously (Mor-Avi, V*. et al.,* "Stability of Albunex® Microspheres under Ultrasonic Irradiation; an *in vitro* Study", *J*. *Am. Soc. Echocardiology* **7**:S29, 1994) was used. This system contains a circular scanning chamber connected to a Masterflex® flow system (Microgon, Inc., Laguna Hills, CA). The scanning chamber was enclosed on each side by water-filled chambers and bound on each side by acoustically transparent material. The PS-ODN-labeled PESDA microbubbles (0.1 milliliters) were injected as a bolus over one second, proximal to the scanning chamber, and then flowed through plastic tubing into the tap water-filled scanning chamber at a controlled flow rate of 100 ml/min. As the bubbles passed through the scanning chamber, the scanner (2.0 MHz frequency, 1.2 MPa peak negative pressure) was set to deliver ultrasound at a conventional 30 Hertz frame rate. Control runs were also made in which no ultrasound was delivered.

Following passage through the scanning chamber, the solution was passed through the same size plastic tubing into a graduated cylinder. The first 10 mL was discarded. The next 10 mL was collected and allowed to stand in order to separate microbubbles on the top from any free oligonucleotide existing in the lower portion of the sample.

Drops from both the upper and lower operation of the effluent were placed upon a hemocytometer slide and analyzed using a 10X magnification. Photographs of these slides were made, and the number of microbubbles over a 36 square centimeter field were hand-counted. Upper and lower portions of the effluents were also mixed 15(v/v) with a solution of formamide and EDTA and heated to 95°C for 5 minutes. These samples were then examined on an Applied Biosystems Model 373A DNA sequencer with a 20% polyacrylamide gel. The data were acquired with GeneScanner® software so that fluorescence intensity area under the curve could be determined.

**Table 3:**

| **Analysis of PS-ODN and Microbubble Counts after Ultrasound Exposure** **(Microbubble Counts in Parentheses)** | | | | | | |
|---|---|---|---|---|---|---|
| | **PS-ODN concentration** **(microbubble count)** | | | | | |
| | **no ultrasound** | | | **with ultrasound** | | |
| **ODN, nM** | **top**^{**a**} | **bottom** | **T/B ratio** | **top** | **bottom** | **T/B ratio** |
| 0.015 | 189 | 158 | 1.2 | 188 | 187 | 1.0 |
| | (112) | (16) | (7.0) | (56) | (12) | (4.7) |
| 0.10 | 169 | 94 | 1.8 | 184 | 191 | 1.0 |
| | (256) | (8) | (8.0) | (16) | (32) | (2.0) |
| 1.0 | 209 | 198 | 1.1 | 254 | 255 | 1.0 |
| | (288) | (8) | (36) | (88) | (40) | (2.0) |
| Mean ± S.E. | 203±19 | 147±25 | 1.5±0.2 | 210±18.6 | 219±20.0 | 1.0±0.0 |
| | (219±54) | (19±7) | (17±10) | (53±21) | (20±10) | (3±1.0) |

| | | | | | | |
|---|---|---|---|---|---|---|
| ^{a}top = top layer of effluent solution after sonification; bottom = bottom layer after sonification | | | | | | |

There is a significant loss of microbubbles in the top layer following exposure to ultrasound (219±54 microbubbles without ultrasound versus 53±21 microbubbles in the top layer of the effluent when exposed to diagnostic ultrasound). This loss of microbubble count was evident regardless of the initial PS-ODN concentration in the upper bubble-containing layer. The PS-ODN concentration in the lower non-bubble containing layer, measured by gel electrophoresis, was significantly increased. The data show that, while exposure of PS-ODN-labeled PESDA microbubbles to diagnostic ultrasound does not alter the integrity of the PS-ODN, it does release it from its albumin binding.

### Example 9. Altered Biodistribution Via Microbubble Delivery of Antisense Oligos

Antisense phosphorothioate oligonucleotides to the cytochrome P450 IIB1 gene sequence were designed, to alter the metabolism of hexobarbital. (PB stimulates CYP IIB1 mRNA, and as a result, hexobarbital, which is hydroxylated by CYP IIB1, is more quickly metabolized and its sedative effect reduced. The antisense oligos are expected to counteract this effect.)

The oligonucleotides were synthesized according to the rat cytochrome P450 IIB1 known sequence and had the following sequences: GGA GCA AGA TAC TGG GCT CCA T (SEQ ID NO:5) and AAA GAA GAG AGA GAG CAG GGA G (SEQ ID NO:6). The oligos were conjugated to perfluoropropane exposed sonicated dextrose/albumin microbubbles (PESDA) as earlier described and delivered to rats intravenously. Male Sprague-Dawley rats (Sasco, Omaha) weighing between 210 to 290 grams were used for all studies. They were housed in animal quarters at the University of Nebraska Medical Center, AAALAC approved animal resource facility. The animals were exposed to 12 hour light/dark cycle and allowed access to Purina rat chow and tap water ad libitum.

Rats in designated groups were injected intraperitoneally with phenobarbital (PB) (Mallinckrodt, St. Louis) at 80 mg/kg/day x 2 days. The PB injections were given simultaneously with the ODN-microbubble injections. Phosphorothioate ODN injections were 1 mg/kg/day x 2 days. Sleep times were measured 48 hours after the first injection.

Each rat was injected with 100 mg/kg of hexobarbital (Sigma, St. Louis) intraperitoneally at the end of 2 days of treatment with PB and/or the ODNs. The animals were placed on their backs to insure that they were still under sedation from the hexobarbital; sleep time is defined as the time they are placed on their backs to the time when they roll over.

Results indicate that delivery of the oligonucleotide conjugated microbubbles greatly improved efficacy of the drug. Control rats had a sleep time of about 23 minutes. Administration of PB reduced sleep time to 11,4±4.5 minutes. Rats given 1/20th dose of microbubble conjugated oligo, however, experienced a sleep time of more than 50 minutes. In comparison, administration of non microbubble conjugated oligo gave little change in sleep time (about 13 minutes).

To determine biodistribution of the oligos, the animals were ultimately sacrificed using ethyl ether, and microsomes were prepared as described by. Franklin and Estabrook (1971). Livers were perfused with 12 ml of 4% saline via the portal vein and then removed from the animal. The livers were minced, homogenized in 0.25 M sucrose (Sigma) and centrifuged at 8000 x g for 20 minutes at 4°C in a Sorvall RC2-B centrifuge (Dupont, Wilmington, DE). The supernatant was saved, resuspended in 0.25 M sucrose and centrifuged at 100,000 x g for 45 minutes at 4°C in a Sorvall OTD55B ultracentrifuge (Dupont). The pellet was resuspended in 1.15 % KCI (Sigma) and centrifuged at 100,000 x g for 1 hour at 4°C, The final pellet was resuspended in an equal volume buffer (10 mM Tris-acetate, 1 mM EDTA, 20% glycerol; Sigma) and frozen at -80°C.

Protein concentrations were determined by Bradford assay (Bradford, 1976). Aliquots of homogenate (80 µl) were added to a 96 well plate (Becton, Dickinson Labware, Lincoln Park, NJ). Bradford reagent (20 µl; Bio-Rad, Richmond, CA) was then added and the plates read at 595 nm on a microplate reader (Molecular Devices, Newport MN). The data was compared to a standard curve generated with known concentrations of bovine serum albumin (Sigma).

CYP IIB1 content was determined by pentoxyresorufin O-dealkylation (PROD) activity (see e.g. Lubet, R.A. *et al., Arch. Biochem. Biophys.* **238**(1):43-8, 1985). For each microsomal sample, 1 mg protein in 1 ml 0.1 M potassium phosphate buffer, 1 ml 2 µM 5-pentoxyresorufin (Pierce, Rockford, IL), and 17 µl 60 mM NADPH were mixed and incubated for 10 minutes at 37°C. The mixture was then added to a 2 ml cuvette and read on a RF5000U spectrofluorophotometer (Shimadzu, Columbia, MD) using an excitation wavelength of 530 nm and emission wavelength of 585 nm. Concentrations of unknowns were calculated from a standard curve of resorufin (Pierce, Rockford, IL) standards. Results were recorded in nmol resorufin/mg protein/min.

Direct measurement of CYP IIB1 protein was determined by an ELISA assay using an antibody directed to the CYP IIB1 protein (Schuurs and Van Weeman, *Clin. Chim. Acta* **81**(1): 1-40, 1977). Liver samples (50 µg per well) were plated in 100 µl 0.35% sodium bicarbonate buffer overnight on a 96 well nunc-immuno plate (InterMed, Skokie, IL). The microsomes were washed 3x with 1 % bovine serum albumin in PBS (PBS/BSA) and incubated for 1 hr at 37°C with 200 µl PBS/BSA. The PBS/BSA was removed, and 50 µl of CYP IIB1 antibody (Oxygene, Dallas, TX) was added and incubated for 1 hour at 37°C. The microsomes were washed 5x with saline/Tween® 20 (Sigma), and 50 µl horseradish peroxidase antibody (Bio-Rad) was added. The microsomes were incubated for 1 hour at 37°C and washed 5x with saline/Tween® and twice with 85% saline. Horseradish peroxidase substrate (100 µl; Kirkegaard & Perry Labs, Gaithersburg, MD) was added and the plate read continuously in a microplate reader (Molecular Devices) at 405 nm for 1 hour. Results were recorded as horseradish peroxidase activity in mOD/min.

Results demonstrated that the oligo conjugated microbubbles directed the oligo to the liver and kidney, sites of phenobarbital metabolism.

### Example 10. Size and Concentration of PESDA Microbubbles after Sonication in Normal and Oxygen-Rich Arterial Blood

Arterial blood was taken from four dogs and three healthy pigs under conditions of room air inhalation just prior to sacrifice. In four of the animals, additional arterial blood was obtained after the animal had inhaled 100% oxygen for a minimum of 10 minutes. The blood was collected in 60 ml heparinized syringes and kept in a warm water bath at 37°C until injected into the scanning chamber. Immediately before injection of the blood into the scanning chamber, 0.2 ml of perfluorocarbon containing microbubbles (PCMB) were injected via a stopcock into the 60 ml syringe of blood, and mixed gently by inverting and rolling the syringe by hand.

The sample was then injected into the scanning chamber at a flow rate of 50 ml/minute. Once the chamber was filled, a closed stopcock connecting the scanning chamber to the plastic tubing used for injection was opened, and ultrasound exposure (intermittent at 1 Hz frame rate or conventional at 30-45 Hz) was initiated. After ultrasound exposure, the effluent blood flowed out of the scanning chamber into tubing which was connected to a graduated cylinder. The first 10 ml of blood was discarded, and the next 15 ml of blood was collected in three 5 ml aliquots into inverted capped syringes. Three minutes following the collection of the last 5 ml sample, a tuberculin syringe was dipped into the top level of the effluent blood and a drop placed on a hemocytometer slide; this length of time was chose to allow the microbubbles in the effluent blood to rise to the top and be collected. The hemocytometer slide was then examined at 40X magnification with a light microscope (Olympus BH-2, Olympus America Inc., Woodbury, NY) and the field containing the highest concentration of microbubbles was photographed on the hemocytometer field.

The photos were later enlarged, and a 25 cm² field was chosen to analyze concentration and mean diameter of the microbubbles in the field. Concentration was determined by counting the total number of bubbles in the entire slide. Microbubble concentration measured with this technique has correlated very closely with Coulter counter measurements, and size measurements with this technique have been calibrated with a known 5 micron sphere (Coulter Size Standards Nominal 5 µm Microspheres, Miami, FL).

*In Vitro* Scanning Chamber: The scanning chamber system consisted of a 35 ml cylindrical scanning chamber connected to a peristaltic Masterflex flow system (Microgon, Inc., Laguna Hills, CA). Enclosed on both sides of the scanning chamber are cylindrical saline filled chambers, bound by acoustically transparent latex material 6.6 microns in thickness (Safeskin, Inc.; Boca Raton, FL). Pressure within the scanning chamber during ultrasound exposure was measured with a pressure transducer placed proximal to the scanning chamber (model 78304A; Hewlett Packard Co., Andover, MA), and averaged 7±3 mm Hg throughout all of the trials.

Two different 2.0 Megahertz ultrasound transducers were used for the *in vitro* studies (Hewlett Packard 1500; Andover, Massachusetts; and HDI 3000, Advanced Technology Laboratories, Bothell, Washington). The peak negative pressure generated was 1.1 MPa for the Hewlett Packard transducer and 0.9 MPa for the HDI 3000 scanner. Imaging depth for all studies was 8.2 centimeters, and the focal point for both transducers was 8 centimeters. The frame rate for each transducer was either conventional (30-42 Hz) or intermittent (1 Hz). All images from the scanning chamber were recorded on high fidelity videotape.

Results: Table 3 demonstrates differences in mean microbubble size for PCMB after exposure to ultrasound in arterial blood (room air and 100% oxygen). When PCMB were exposed to 100% oxygenated arterial blood, there was a significant decrease in mean microbubble size after insonation (p=0.01). The smaller microbubble size was seen both after intermittent imaging (7.3±3.7 µm room air vs. 6.4±3.2 µm 100% oxygen) and after conventional imaging (7.5±3.5 µm room air vs. 6.3±3.0 µm 100% oxygen).

Microbubble concentration decreased significantly after exposure to conventional frame rates when compared to intermittent imaging in room air arterial blood (Table 3). However, conventional frame rates at the same transducer output did not destroy as many PCMB when they were in oxygenated arterial blood.

**Table 4:**

| **Comparison of Effluent PESDA Microbubble Size After Exposure to Ultrasound in Normal (Room Air) and Oxygenated Arterial Blood** | | | |
|---|---|---|---|
| | | MB Conc. (No/hpf) | |
| | MB size, µm | Conv | Inter |
| Arterial | 7.4±3.6 | 6 ± 8 | 16 ± 11^{a} |
| Arterial + O₂ | 6.3±3.1 | 11 ± 9 | 14 ± 9^{b} |

| | | | |
|---|---|---|---|
| No./hpf = Number of microbubbles per high-power field Conv = Conventional frame rates (80 to 43 Hz) Inter = Intermittent imaging at 1 Hz ^{a}p<0.05 compared with arterial conv. | | | |
| ^{b}p < 0.05 r test compared with arterial samples | | | |

Microbubble concentration decreased significantly after exposure to conventional frame rates when compared to intermittent imaging in room air arterial blood (Table 4). However, conventional frame rates at the same transducer output did not destroy as many PCMB when they were in oxygenated arterial blood.

### Example 11. in vivo Imaging Study of Microbubbles Formed in N₂-Free Versus Room Air Environments

Perfluorocarbon containing microbubbles (PCMB) were prepared as described in Example 1. One part 5% human serum albumin and three parts 5 % dextrose (total 16 ml) were hand-agitated with 8 ml of decafluorobutane. Following the agitation, the sample underwent electromechanical sonication for 80±2 seconds. The 80 second sonication process was performed in two different environments: either room air (RA) or 100% oxygen (N₂-free environment) was blown into the interface between the sonicating horn and perfluorocarbon dextrose/albumin solution during sonication.

*In Vivo* Imaging: Methods of diagnostic ultrasound imaging, in which microbubbles, formed by sonicating an aqueous protein solution, are injected into a mammal to alter the acoustic properties of a predetermined area which is then ultrasonically scanned to obtain an image for use in medical procedures, are well known. For example, see U.S. Patent Nos.4,572,203, 4,718,433 and 4,774,958.

Samples of microbubbles exposed to either 100% oxygen (O₂ PCMB) or room air (RA PCMB) during sonication were injected intravenously in dogs. Imaging was performed with a 1.7 MHz harmonic transducer (HDI 3000; Advanced Technology Laboratories; Bothell, Washington). Transducer output was set to 0.3-0.8 MPa, and kept constant for all comparisons of videointensity from the two different microbubble samples. Frame rates for comparison of background subtracted myocardial videointensity were either 43 Hz (conventional) or 10 Hz (intermittent). All procedures were approved by the Institutional Animal Care and Use Committee and was in compliance with the Position of the American Heart Association on Research Animal Use.

The bolus injections of RA (room air) PCMB and O₂ PCMB were either 0.0025 or 0.005 ml/kg. The concentration of microbubbles in the samples were determined to be the same. Peak anterior and posterior myocardial videointensity were measured from digitized super VHS videotape images (Maxell, Japan) obtained off-line using a Tom-Tech review station (Louisville, Colorado), which quantifies videointensity over a 1-255 gray scale range. The region of interest was placed in the mid myocardium of each segment.

In addition to this quantitative analysis, a visual assessment of regional myocardial contrast enhancement in the anterior, septal, lateral, and posterior regions from the short axis view was made by two independent reviewers. Each region was assigned a 0 (no myocardial contrast), 1 + (mild myocardial contrast enhancement) or 2+ (bright myocardial contrast enhancement which approached cavity intensity).

A total of six comparisons of peak myocardial videointensity between O₂ PCMB and RA PCMB were made in the three dogs. In Table 5, it can be seen that prior to injection, the PCMB sonicated in the presence of 100% oxygen were similar in size and concentration to PCMB sonicated in the presence of room air. However, in all three dogs, the peak myocardial videointensity using the 10 Hertz frame rate (intermittent imaging) was significantly higher for the PCMB sonicated in the presence of 100% oxygen.

Only the oxygenated PCMB produced a consistent homogenous myocardial contrast at the doses used for transthoracic imaging. Visual myocardial contrast was 2+ in 20 of the 24 regions following intravenous O₂ PCMB injections compared to 9 or 24 regions following the same dose of RA PCMB (p=0.001).

**Table 5:**

| **Comparison of PMVI (Peak Myocardial Videointensity) After IV Injection of Fluorocarbon-Containing PCMB Sonicated in 100% Oxygen or Room Air** | | | | |
|---|---|---|---|---|
| | PMVI (units) | | Microbubble | |
| | Ant | Post | Size (µm) | Conc (No./hpf) |
| Room Air PCMB | 54 ± 12 | 19 ± 9 | 4.0 ± 2.4 | 109 ± 30 |
| O₂ PCMB | 70 ± 6^{a} | 31 ± 5^{a} | 3.9 ± 2.3 | 108 ± 50 |

| | | | | |
|---|---|---|---|---|
| Ant = anterior myocardium; Post = posterior myocardium Conc = MB concn immediately after sonication; No./hpf = number of MB per high-power field ^{a}p<0.05 compared with RA PCMB | | | | |

Only the oxygenated PCMB produced a consistent homogenous myocardial contrast at the doses used for transthoracic imaging. Visual myocardial contrast was 2+ in 20 of the 24 regions following intravenous 0₂ PCMB injections compared to 9 or 24 regions following the same dose of RA PCMB (p=0.001).

As expected, microbubble concentrations in room air blood were significantly reduced when exposed to higher frame rates. However, this destruction by more rapid frame rates was attenuated somewhat when the PCMB were in oxygenated blood. The reason for this difference is unclear. One possibility is that the more rapid diffusion of nitrogen out of the microbubbles in oxygenated blood created a higher internal concentration of perfluorocarbon, and thus increased the diffusion gradient for the insoluble perfluorocarbon. Due to its low solubility, its enhanced diffusion out of the microbubble would lead to the formation of smaller unencapsulated perfluorocarbon microbubbles. The hemocytometry resolution would be unable to differentiate encapsulated from unencapsulated microbubbles and thus would count them both. This explanation may also account for the smaller mean microbubble size observed for PCMB exposed to 100% oxygenated arterial blood.

Statistical Analysis: An unpaired t test was used to compare microbubble size and concentration of the PCMB samples exposed to different gases during sonications. This was also used to compare differences in peak myocardial videointensity in the *in vivo* studies. If the data was not normally distributed, a non-parametric test was performed. Comparisons of visual myocardial contrast enhancement following intravenous O₂ PCMB and RA PCMB were made with continency tables (Fisher's Exact Test). A p value less than 0.05 was considered significant.

A coefficient of variation was used to measure interobserver variability in the measurements of microbubble size and concentration in the *in vitro* studies. A mean difference between independent reviewers was used to compare interobserver variations in peak myocardial videointensity.

Two independent observers measured microbubble size and concentration of six different slides exposed to either intermittent or conventional ultrasound frame rates. The coefficient of variation for measurements of microbubble size by two independent observers in six different samples was 8 % (r=0.95; p=0.004), while the coefficient of variation for independent measurements of microbubble concentration was 9% (r=0.99; p<0.001). The reported mean difference in peak myocardial videointensity measurements by two independent reviewers for transthoracic imaging is 4±4 units (r=0.94, SEE=5 units; p<0.001; n=24 comparisons), which is well below the 16 unit mean difference in anterior and 13 unit mean difference in posterior peak myocardial videointensity between 02 PCMB and RA PCMB. The two investigators were in agreement of the visual degree of contrast enhancement in 37 of the 44 regions (84%). Five of the discrepancies were in visual grading of RA PCMB myocardial contrast enhancement (0 vs 1 + in two regions, I + vs. 2 + in three regions). The three regions where there was disagreement on whether there was 1 + vs 2 + were assigned a 2 + in the statistical analysis.

### Example 12. Effect of Targeted Sonification on Uptake of ODN from Microbubbles

An *in vivo* study of the effect of sonification on kidney uptake of PS-ODN conjugated microbubbles was performed in three dogs. Intravenous injections of fluorescently labeled PS-ODN PESDA microbubbles (0.2 ml) were given in the femoral vein. At the same time, the left kidney was insonified by an externally placed 2.0-2.5 MHz diagnostic ultrasound transducer (peak negative pressure 1.1 MPa). The kidney was insonified using a 30 Hz frame rate for a minimum of 2 minutes after injection, and during the visually evident appearance of contrast in the kidney. In each dog, left ventricular and pulmonary artery pressures were monitored before and after kidney injection using saline filled catheters placed in the left ventricle and pulmonary artery, respectively. Approximately four hours after injection, the dog was sacrificed and both kidneys removed. Cut sections were obtained from the renal cortex and sampled for PS-ODN, counted using the gene scanning methods described above (Example 8). Histologic sections were also obtained for the analysis of fluorescence in the glomeruli and nephrons.

In one dog, there was over a 10-fold greater uptake of PS-ODN in the insonified kidney compared to the nbninsonified kidney following intravenous PS-ODN in labeled PESDA. In two of the three dogs, this partitioning of PS-ODN uptake to the insonified kidney was evident. In the first dog, there was a 3-fold higher uptake of PS-ODN in the insonified kidney versus the noninsonified kidney. In the second dog, there was over a nine-fold higher uptake of PS-ODN in the insonified kidney. In the third dog, however, there was no difference in PS-ODN uptake between the two kidneys. There were no hemodynamic changes following the intravenous injections of PS-ODN-labeled PESDA microbubbles. Histologic examination of the kidneys post-mortem also indicated no destruction of any glomerular or tubular cells by the diagnostic ultrasound.

### Example 13. Effect of Sonification on Uptake of Heparin from Microbubbles

Intravenous heparin was administered to a canine subject, in a bolus dose of 1500 units, in three different settings. In setting 1, heparin was given as the free drug. In setting 2, 1500 units heparin was given bound to orosomucoid PESDA, but no ultrasound was applied to the blood pool. In setting 3, the same dose of heparin (1500 units) bound to orosomucoid PESDA was given, and ultrasound was applied to the blood pool. Baseline measurements of activated partial thromboplastin time (PTT) were measured and then repeated at five minute intervals after each injection for a minimum of 15 minutes.

In setting 1, the PTT increased to > 106 seconds at five minutes, but returned to 30 - 60 seconds at 15 minutes. In setting 2, the PTT reached > 106 seconds after 10 minutes, and returned to 60 - 80 seconds at 15 minutes. In setting 3, the PTT remained at > 106 seconds after 15 minutes; no further measurements were made.

### Example 14. Inhibition of Carotid Artery Neointimal Formation using Transcutaneous Ultrasound and an Intravenous Microbubble Delivery System Containing Antisense to the c-myc Protooncogene

All procedures were approved by the Institutional Animal Care and Use Committee and were in compliance with the Position of the American Heart Association on Research Animal Use. Twenty eight domestic pigs were premedicated with aspirin (325 mg PO). General anesthesia was then administered using ketamine (20 mg/kg), xylazine (4 mg/kg) and supplemental pentobarbital. The pigs were intubated and placed on a respirator breathing room air. A Swan Ganz catheter was advanced into the pulmonary artery to monitor pulmonary pressure. Intravenous heparin (4,000-5,000 units), atropine (0.5-1.0 mg), and sublingual nifedipine (10-30 mg) were given. An 8F guide catheter was placed into the proximal portion of the right carotid artery. The artery was injured by dilating the vessel with an oversized balloon (6.0 mm to 10.5 mm) for a mean of 107±34 seconds (range 90-240 seconds). The intervention was performed by an experienced interventional cardiologist (U.D.) who had no knowledge of which treatment the pig was to subsequently receive. Vessel patency following injury was confirmed angiographically with intracarotid injections of Hexabrix or Renographin-76.

The first 20 animals were randomized to receive one of three treatments following balloon injury:
(a) intravenous phosphorothioate ODN to c-*myc* (0.5 milligrams; Lynx Therapeutics; Hayward, California) bound to PESDA (ODN-PESDA);
(b) the same dose of intravenous antisense to the c-*myc* alone (ODN alone); or
(c) no injections (control).

Injections were repeated on day three following balloon injury in ODN-PESDA and ODN alone pigs. All animals received Ketorolac (60 mg) and Solumedrol (40 mg) intravenously to prevent pulmonary hypertensive responses in those pigs randomized to receive microbubbles.

The last eight pigs received a different ODN to *c-myc* (Morpholino; AVI Biopharma, Inc; Corvallis, Oregon). In these pigs, the first four received ODN bound to PESDA, and the last four received either ODN alone (n=2) or no treatment (n=2) following balloon injury.

In those animals randomized to ODN-PESDA, a transcutaneous 20 kilohertz ultrasound probe (Model XL2020; Heat Systems; Farmingdale, New York) at a power output of 50 watts/cm² insonified the region over the right carotid artery before and after each injection for a total of six minutes both immediately after balloon dilatation and on day three. To avoid skin irritation due to probe heating, a 1.5-2.0 centimeter coupling gel was placed between the skin surface and the probe tip using an inverted cut-off 12 milliliter syringe. The right carotid artery location was confirmed with a diagnostic transducer (HDI3000; Advanced Technology Laboratory; Bothell, Washington).

Measurements at 30 days Following Balloon Injury: At 30 days following balloon injury, intravascular ultrasound (IVUS) measurements were made prior to sacrificing the pig. A 2.9 or 3.1 F 30 Megahertz IVUS catheter (CVIS; Sunnyvale, CA) was advanced under fluoroscopy into the distal carotid artery and a motorized pullback of the catheter was performed. Off line planimetry of lumen area, total vessel area (lumen plus any visualized plaque), and vessel diameter was performed at the site of greatest plaque and smallest lumen in the previously injured area. The pigs were then sacrificed, and serial sections of the carotid artery performed after fixation. The site of maximal intimal thickness was measured by digital planimetry (NIH Image 1.61; Bethesda, Maryland). Since the vessels did not undergo perfusion fixation, the value for maximal intimal thickness was indexed to vessel diameter at the balloon injury site measured by IVUS. Both IVUS and histologic measurements were made by reviewers (W.H. and S.R, respectively) who had no knowledge of which treatment regimen the pig received.

Statistical Analysis: Differences in IVUS measurements of total vessel area, lumen area, and lumen diameter at the balloon injury sites and histologic measures of maximal intimal thickness in the three groups were compared using analysis of variance (Student-Newmann-Keuls multiple comparison procedures). Since IVUS could not be performed in three pigs receiving ODN alone, data from ODN alone and control were also combined and compared with ODN-PESDA by Student's t-test or Mann-Whitney Rank Sum test. Interobserver variability in IVUS and histologic measurements were computed by having the same reviewer repeat measurements at different times and compute the percent difference between measurements.

Results: Two pigs died during the balloon injury protocol. In five pigs, (three ODN-PESDA, one IV ODN alone, and one control) there was no histologic evidence of injury or intimal hyperplasia at 30 days following injury. Of the remaining 21 pigs, eight received ODN-PESDA, seven received intravenous ODN alone, and six received nothing. The pigs treated with 20 kilohertz ultrasound had a superficial abrasion at the site of applied ultrasound which was no longer evident at 30 days follow-up.

Table 1 contains the intravascular ultrasound and histologic data in the three groups. Intravascular ultrasound was possible in 16 of the pigs. It could not be performed in three of the pigs that received ODN alone because of thrombotic occlusion at 30 days following injury, and in one each of the pigs that received ODN-PESDA and ODN alone for technical reasons.

Both total vessel area and lumen area at the injury site measured with IVUS were significantly larger in the ODN-PESDA group. This larger vessel size was seen despite significantly smaller maximal intimal thickness in this same group at histology. When histologic measures of maximal intimal thickness were indexed to IVUS vessel diameter, there was a clear cut distinction between the three groups (Table 1).

The control pig had a smaller vessel but greater intimal thickness at 30 days when compared to the pig treated with ultrasound and ODN-PESDA. Interobserver variability was 3% (n =12 comparisons) in IVUS measurements, and 14% (n=25 comparisons) for repeated histologic measurements.

**Table 6.**

| **Intravascular Ultrasound (IVUS) and Histology Findings 30 Days Following Carotid Balloon Injury in Pigs Receiving IV ODN-PESDA with Transcutaneous Ultrasound, ODN Alone, or No Treatment (Control)** | | | | |
|---|---|---|---|---|
| **Treatment** | **MIT (mm)** | **MIT Index** | **LA (mm**^{**2**}**)** | **TVA (mm**^{**2**}**)** |
| ODN-PESDA | 0.14±0.04^{a} | 0.58±0.22^{b} | 21±3^{c} | 24±4^{c} |
| ODN alone | 0.41±0.34 | 1 .26±0.45 | 13±5 | 15±4 |
| Control | 0.22±0.09 | 1.66±0.86 | 13±6 | 15±5 |

| | | | | |
|---|---|---|---|---|
| MIT = maximal intimal thickness (histology); MIT Index=MIT indexed to vessel diameter (IVUS) LA = lumen area (IVUS); TVA=total vessel area (IVUS) ^{a}p<0.05 compared to ODN alone | | | | |
| ^{b}p=0.01 compared to ODN alone and control combined | | | | |
| ^{c}p<0.05 compared to all other groups | | | | |

## Claims

1. Use of an aqueous suspension of a plurality of protein-encapsulated, insoluble gas-containing microbubbles for the preparation of a medicament for delivering a biological agent to a specific tissue site,
wherein said protein is conjugated to the biological agent, and said microbubbles are formed under conditions which produce an enhanced oxygen content and a lower partial pressure of N₂ within the microbubbles than that obtained via room air sonication,
which medicament is to be administered to an animal, such that said protein directs the microbubble-conjugated agent to sites of bioprocessing of said protein and, upon dissipation of the microbubble, releases said agent.

2. The use of claim 1, wherein dissipation of the microbubble is effected by exposing the tissue site to ultrasound.

3. Use of an aqueous suspension of a plurality of protein-encapsulated, insoluble gas-containing microbubbles for the preparation of a medicament for delivering a biological agent to a target site in an animal,
wherein said protein is conjugated to the biological agent, and said microbubbles are formed under conditions which produce an enhanced oxygen content and a lower partial pressure of N₂ within the microbubbles than that obtained via room air sonication,
whereby after the administration of said suspension to the animal an ultrasound field at the target site is to be applied.

4. The use of any of claims 1 to 3, wherein said microbubbles are formed in an N₂-free environment.

5. The use of claim 4, wherein said environment consists of oxygen.

6. The use of any of claims 1 to 3, wherein said protein is selected from the group consisting of albumin, human gamma globulin, human apotransferrin, β-lactose and urease.

7. The use of claim 6, wherein said protein is albumin.

8. The use of any of claims 1 to 3, wherein said insoluble gas is selected from the group consisting of perfluoromethane, perfluoroethane, perfluoropropane, perfluorobutane, and perfluoropentane.

9. The use of any of claims 1 to 3, wherein said biological agent is a polynucleotide, selected from the group consisting of an antisense oligonucleotide, an antigen oligonucleotide, an oligonucleotide probe, a vector, a viral vector and a plasmid.

10. The use of any of claims 1 to 3, wherein said microbubbles are formed by:
(a) diluting an aqueous albumin solution comprising about 2% to about 10% by weight of human serum albumin by about two-fold to about eight-fold with an aqueous dextrose solution comprising 5% to 50% by weight of dextrose;
(b) agitating said solution with said insoluble gas; and
(c) exposing said solution to a sonication horn in an N₂-deplered environment to create cavitation at particulate sites in said solution, thereby generating stable microbubbles from about 0.1 to 10 microns in diameter.

11. The use of claim 10, wherein in step (c) said N₂-depleted environment consists of oxygen.

12. The use of claim 11, wherein the oxygen is blown into an interface between the sonicating horn and the solution.

13. Use of an aqueous suspension of a plurality of protein-encapsulated, insoluble gas-containing microbubbles for the preparation of a medicament for preventing carotid or coronary artery stenosis formation in an animal undergoing vascular trauma,
wherein said protein is conjugated to an oligonucleotide which inhibits the expression of a gene which encodes a regulatory enzyme which mediates smooth muscle proliferation, and said microbubbles are formed under conditions which produce an enhanced oxygen content and a lower partial pressure of N₂ within the microbubbles than that obtained via room air sonication, whereby the administration of the medicament is to be followed by the application of an ultrasonic field at the artery site.

14. The use of claim 13, wherein said gene is c-myc or c-myb.

15. A microbubble composition, comprising an aqueous suspension of protein-encapsulated insoluble gas-containing microbubbles having an enhanced oxygen content and a lower partial pressure of N₂ within said microbubbles than that of room air-sonicated microbubbles.

16. The composition of claim 15, further comprising a biological agent conjugated to said protein.

17. The composition of claim 15, wherein said protein is selected from the group consisting of albumin, human gamma globulin, human apotransferrin, β-lactose and urease.

18. The composition of claim 17, wherein said protein is albumin.

19. The composition of claim 15, wherein said insoluble gas is a perfluorocarbon.

20. The composition of claim 19, wherein said gas is selected from the group consisting of perfluoromethane, perfluoroethane, perfluoropropane, perfluorobutane, and perfluoropentane.

21. The composition of claim 16, wherein said biological agent is an oligonucleotide.

## Patentansprüche

1. Verwendung einer wässrigen Suspension aus einer Vielzahl von mit Proteinen umhüllten, unlösliches Gas enthaltenden Mikrobläschen für die Herstellung eines Medikaments zur Bereitstellung eines biologischen Mittels an einer bestimmten Stelle im Gewebe,
wobei das Protein an das biologische Mittel konjugiert ist und die Mikrobläschen unter Bedingungen gebildet werden, die zu einem erhöhten Sauerstoffgehalt und einem niedrigeren Partialdruck von N₂ innerhalb der Mikrobläschen führen als durch Beschallung mit Raumluft erreicht wird;
wobei das Medikament einem Tier verabreicht werden soll, so dass das Protein das Mikrobläschen-konjugierte Mittel an die Stellen der Bioprozessierung des Proteins weiterleitet und nach Dissipation des Mikrobläschens dieses Mittel freisetzt.

2. Verwendung nach Anspruch 1, wobei die Dissipation des Mikrobläschens dadurch erreicht wird, dass die Gewebestelle Ultraschall ausgesetzt wird.

3. Verwendung einer wässrigen Suspension aus einer Vielzahl von mit Proteinen umhüllten, unlösliches Gas enthaltenden Mikrobläschen für die Herstellung eines Medikaments zur Bereitstellung eines biologischen Mittels an einer Zielstelle in einem Tier,
wobei das Protein an das biologische Mittel konjugiert ist und die Mikrobläschen unter Bedingungen gebildet werden, die zu einem erhöhten Sauerstoffgehalt und einem niedrigeren Partialdruck von N₂ innerhalb der Mikrobläschen führen als durch Beschallung mit Raumluft erreicht wird,
wobei nach Verabreichung der Suspension an das Tier ein Ultraschallfeld an der Zielstelle angelegt werden soll.

4. Verwendung nach einem der Ansprüche 1 bis 3, wobei die Mikrobläschen in einer N₂-freien Umgebung gebildet werden.

5. Verwendung nach Anspruch 4, wobei die Umgebung aus Sauerstoff besteht.

6. Verwendung nach einem der Ansprüche 1 bis 3, wobei das Protein ausgewählt ist aus der Gruppe bestehend aus Albumin, menschlichem Gammaglobulin, menschlichem Apotransferrin, β-Lactose und Urease.

7. Verwendung nach Anspruch 6, wobei das Protein Albumin ist.

8. Verwendung nach einem der Ansprüche 1 bis 3, wobei das unlösliche Gas ausgewählt ist aus der Gruppe bestehend aus Perfluormethan, Perfluorethan, Perfluorpropan, Perfluorbutan und Perfluorpentan.

9. Verwendung nach einem der Ansprüche 1 bis 3, wobei das biologische Mittel ein Polynucleotid ist, das ausgewählt ist aus der Gruppe bestehend aus einem Antisense-Oligonucleotid, einem Antigen-Oligonucleotid, einer Oligonucleotid-Sonde, einem Vektor, einem viralen Vektor und einem Plasmid.

10. Verwendung nach einem der Ansprüche 1 bis 3, wobei die Mikrobläschen gebildet werden durch:
(a) Verdünnen einer wässrigen Albuminlösung, die etwa 2 Gew.-% bis etwa 10 Gew.-% menschliches Serumalbumin enthält, um das etwa Zweifache bis etwa Achtfache mit einer wässrigen Dextroselösung, die 5 Gew.-% bis 50 Gew.-% Dextrose enthält;
(b) Schütteln der Lösung mit dem unlöslichen Gas; und
(c) Aussetzen der Lösung einem Beschallungstrichter in einer N₂-freien Umgebung, um eine Hohlraumbildung an bestimmten Stellen in der Lösung zu schaffen, wodurch stabile Mikrobläschen mit einem Durchmesser von etwa 0,1 bis 10 Mikron erzeugt werden.

11. Verwendung nach Anspruch 10, wobei die N₂-freie Umgebung in Schritt (c) aus Sauerstoff besteht.

12. Verwendung nach Anspruch 11, wobei der Sauerstoff in eine Grenzfläche zwischen dem Beschallungstrichter und der Lösung geblasen wird.

13. Verwendung einer wässrigen Suspension aus einer Vielzahl von mit Proteinen umhüllten, unlösliches Gas enthaltenden Mikrobläschen für die Herstellung eines Medikaments zur Verhinderung der Bildung einer Karotisstenose oder Koronararterienstenose in einem Tier, das an einem Gefäßtrauma leidet,
wobei das Protein an ein Oligonucleotid konjugiert ist, das die Expression eines Genes hemmt, das ein regulatorisches Enzym codiert, das die Proliferation des glatten Muskels vermittelt, und wobei die Mikrobläschen unter Bedingungen gebildet werden, die zu einem erhöhten Sauerstoffgehalt und einem niedrigeren Partialdruck von N₂ innerhalb der Mikrobläschen führen als durch Beschallung mit Raumluft erreicht wird, wobei das Anlegen eines Ultraschallfelds an der Stelle der Arterie nach der Verabreichung des Medikaments stattfinden soll.

14. Verwendung nach Anspruch 13, wobei das Gen c-myc oder c-myb ist.

15. Mikrobläschenzusammensetzung, umfassend eine wässrige Suspension, die mit Proteinen umhüllte, unlösliches Gas enthaltende Mikrobläschen umfasst, die einen erhöhten Sauerstoffgehalt und einen niedrigeren Partialdruck von N₂ innerhalb der Mikrobläschen besitzen als mit Raumluft beschallte Mikrobläschen.

16. Zusammensetzung nach Anspruch 15, weiterhin umfassend ein biologisches Mittel, das an das Protein konjugiert ist.

17. Zusammensetzung nach Anspruch 15, wobei das Protein ausgewählt ist aus der Gruppe bestehend aus Albumin, menschlichem Gammaglobulin, menschlichem Apotransferrin, β-Lactose und Urease.

18. Zusammensetzung nach Anspruch 17, wobei das Protein Albumin ist.

19. Zusammensetzung nach Anspruch 15, wobei das unlösliche Gas Perfluorcarbon ist.

20. Zusammensetzung nach Anspruch 19, wobei das Gas ausgewählt ist aus der Gruppe bestehend aus Perfluormethan, Perfluorethan, Perfluorpropan, Perfluorbutan und Perfluorpentan.

21. Zusammensetzung nach Anspruch 16, wobei das biologische Mittel ein Oligonucleotid ist.

## Revendications

1. Utilisation d'une suspension aqueuse d'une pluralité de microbulles encapsulées dans une protéine, contenant un gaz insoluble, pour la préparation d'un médicament destiné au transport d'un agent biologique à un site spécifique d'un tissu,
dans laquelle ladite protéine est conjuguée à l'agent biologique et lesdites microbulles sont formées dans des conditions qui produisent une augmentation du contenu en oxygène et une pression partielle en N₂ dans les microbulles inférieure à celle obtenue par une sonication en air ambiant ;
dans laquelle le médicament doit être administré à un animal de telle manière que ladite protéine dirige l'agent conjugué à la microbulle vers les sites de transformation biologique de ladite protéine et, après dissipation de la microbulle, libère ledit agent.

2. Utilisation selon la revendication 1, dans laquelle la dissipation de la microbulle est effectuée en exposant le site du tissu aux ultrasons.

3. Utilisation d'une suspension aqueuse d'une pluralité de microbulles encapsulées dans une protéine, contenant un gaz insoluble, pour la préparation d'un médicament destiné au transport d'un agent biologique à un site cible dans un animal,
dans laquelle ladite protéine est conjuguée à l'agent biologique et lesdites microbulles sont formées dans des conditions qui produisent une augmentation du contenu en oxygène et une pression partielle en N₂ dans les microbulles inférieure à celle obtenue par une sonication en air ambiant ;
par laquelle, après l'administration de ladite suspension à l'animal, un champ d'ultrasons doit être appliqué au site cible.

4. Utilisation selon l'une des revendications 1 à 3, dans laquelle les microbulles sont formées dans un environnement dénué de N₂ libre.

5. Utilisation selon la revendication 4, dans laquelle ledit environnement se compose d'oxygène.

6. Utilisation selon l'une des revendications 1 à 3, dans laquelle ladite protéine est sélectionnée parmi le groupe constitué de l'albumine, la gamma globuline humaine, l'apotransferrine humaine, le β-lactose, et l'uréase.

7. Utilisation selon la revendication 6, dans laquelle ladite protéine est l'albumine.

8. Utilisation selon l'une des revendications 1 à 3, dans laquelle ledit gaz insoluble est sélectionné parmi le groupe constitué du perfluorométhane, du perfluoroéthane, du perfluoropropane, du perfluorobutane, et du perfluoropentane.

9. Utilisation selon l'une des revendications 1 à 3, dans laquelle ledit agent biologique est un polynucléotide, sélectionné parmi le groupe constitué d'un oligonucléotide antisens, d'un oligonucléotide antigène, d'une sonde oligonucléotidique, d'un vecteur, d'un vecteur viral et d'un plasmide.

10. Utilisation selon l'une des revendications 1 à 3, dans laquelle lesdites microbulles sont formées par :
(a) Dilution d'un facteur d'environ deux à environ huit d'une solution aqueuse d'albumine comprenant environ 2 % à environ 10 % en poids d'albumine sérique humaine avec une solution aqueuse de dextrose comprenant 5 à 50 % en poids de dextrose ;
(b) Agitation de ladite solution avec ledit gaz insoluble ; et
(c) Exposition de ladite solution à une corne de sonication dans un environnement déplété en N₂ pour créer une cavitation en des sites particulaires dans ladite solution, générant par cela des microbulles stables d'environ 0,1 à 10 microns de diamètre.

11. Utilisation selon la revendication 10, dans laquelle dans l'étape (c) ledit environnement déplété en N₂ se compose d'oxygène.

12. Utilisation selon la revendication 11, dans laquelle l'oxygène est soufflé dans l'interface entre la corne de sonication et la solution.

13. Utilisation d'une suspension aqueuse d'une pluralité de microbulles encapsulées dans une protéine, contenant un gaz insoluble, pour la préparation d'un médicament destiné à la prévention de la formation de sténose de la carotide ou de l'artère coronarienne chez un animal subissant des traumatismes vasculaires, dans laquelle ladite protéine est conjuguée à un oligonucléotide qui inhibe l'expression d'un gène qui code pour une enzyme régulatrice médiant la prolifération du muscle lisse, et lesdites microbulles sont formées dans des conditions qui produisent une augmentation du contenu en oxygène et une pression partielle en N₂ dans les microbulles inférieure à celle obtenue par une sonication en air ambiant ; par laquelle l'administration du médicament doit être suivie de l'application d'un champ d'ultrasons sur le site artériel.

14. Utilisation selon la revendication 13, dans laquelle ledit gène est c-myc ou c-myb.

15. Composition de microbulles, comprenant une suspension aqueuse de microbulles encapsulées dans une protéine, contenant un gaz insoluble, ayant une teneur en oxygène augmentée et une pression partielle en N₂ dans les microbulles inférieure à celle de microbulles soniquées en présence d'air ambiant.

16. Composition selon la revendication 15, comprenant en outre un agent biologique conjugué à ladite protéine.

17. Composition selon la revendication 15, dans laquelle ladite protéine est sélectionnée parmi le groupe constitué de l'albumine, la gamma globuline humaine, l'apotransferrine humaine, le β-lactose, et l'uréase.

18. Composition selon la revendication 17, dans laquelle ladite protéine est l'albumine.

19. Composition selon la revendication 15, dans laquelle ledit gaz insoluble est un perfluorocarbone.

20. Composition selon la revendication 19, dans laquelle ledit gaz est sélectionné parmi le groupe constitué du perfluorométhane, du perfluoroéthane, du perfluoropropane, du perfluorobutane, et du perfluoropentane.

21. Composition selon la revendication 16, dans laquelle ledit agent biologique est un oligonucléotide.
